(19) Europäisches Patentamt
European Patent Office
Office européen des brevets

(11) EP 1 360 283 B1

(12) EUROPÄISCHE PATENTSCHRIFT

(45) Veröffentlichungstag und Bekanntmachung des Hinweises auf die Patenterteilung:
**14.02.2007 Patentblatt 2007/07**

(21) Anmeldenummer: **02712891.7**

(22) Anmeldetag: **05.02.2002**

(51) Int Cl.:
*C12N 9/58* (2006.01) *C12N 15/52* (2006.01)
*C12N 15/66* (2006.01) *C12N 15/67* (2006.01)
*C12N 15/80* (2006.01) *C12P 21/00* (2006.01)

(86) Internationale Anmeldenummer:
**PCT/EP2002/001144**

(87) Internationale Veröffentlichungsnummer:
**WO 2002/064760 (22.08.2002 Gazette 2002/34)**

# (54) EXPRESSION DER REKOMBINANTEN PROTEINASE K AUS TRITIRACHIUM ALBUM IN HEFE

EXPRESSION OF RECOMBINANT PROTEINASE K FROM TRITIRACHIUM ALBUM IN YEAST

EXPRESSION DE PROTEINASE K RECOMBINEE CONTENUE DANS LE TRITIRACHIUM ALBUM DANS DE LA LEVURE

(84) Benannte Vertragsstaaten:
**AT BE CH CY DE DK ES FI FR GB GR IE IT LI LU MC NL PT SE TR**

(30) Priorität: **09.02.2001 DE 10105911**

(43) Veröffentlichungstag der Anmeldung:
**12.11.2003 Patentblatt 2003/46**

(73) Patentinhaber:
- **Roche Diagnostics GmbH**
  **68305 Mannheim (DE)**
  Benannte Vertragsstaaten:
  **DE**
- **F.HOFFMANN-LA ROCHE AG**
  **4070 Basel (CH)**
  Benannte Vertragsstaaten:
  **AT BE CH CY DK ES FI FR GB GR IE IT LI LU MC NL PT SE TR**

(72) Erfinder:
- **MUELLER, Rainer**
  **82377 Penzberg (DE)**
- **THALHOFER, Johann-Peter**
  **82362 Weilheim (DE)**
- **GEIPEL, Frank**
  **82377 Penzberg (DE)**
- **GLASER, Stephan**
  **82402 Seeshaupt (DE)**
- **HOELKE, Werner**
  **82377 Penzberg (DE)**
- **SCHOEN, Helmut**
  **82377 Penzberg (DE)**
- **KIRSCHBAUM, Thomas**
  **81543 Muenchen (DE)**

(56) Entgegenhaltungen:
**WO-A-88/07581**

- **PENHEITER ALAN R ET AL: "Purification and characterization of a soybean root nodule phosphatase expressed in Pichia pastoris." PROTEIN EXPRESSION AND PURIFICATION, Bd. 14, Nr. 1, Oktober 1998 (1998-10), Seiten 125-130, XP002216461 ISSN: 1046-5928**
- **SAMAL B ET AL: "Cloning and expression of the gene encoding a novel proteinase from Tritirachium album limber." ADVANCES IN EXPERIMENTAL MEDICINE AND BIOLOGY. UNITED STATES 1996, Bd. 379, 1996, Seiten 95-104, XP008009215 ISSN: 0065-2598 in der Anmeldung erwähnt**
- **EBELING W ET AL: "PROTEINASE K FROM TRITIRACHIUM ALBUM LIMBER" EUROPEAN JOURNAL OF BIOCHEMISTRY, BERLIN, DE, Bd. 47, 1974, Seiten 91-97, XP000864618 ISSN: 0014-2956 in der Anmeldung erwähnt**

EP 1 360 283 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

SEQUENCE LISTING

<110> Roche,Diagnostics GmbH

<120> Expression der rekombinanten Proteinase K aus Tritirachium album in Hefe

<130> 5441/00/DE

<140>
<141>

<160> 21

<170> PatentIn Ver. 2.1

<210> 1
<211> 369
<212> PRT
<213> Tritirachium album Limber

<400> 1

Ala Pro Ala Val Glu Gln Arg Ser Glu Ala Ala Pro Leu Ile Glu Ala
1 5 10 15

Arg Gly Glu Met Val Ala Asn Lys Tyr Ile Val Lys Phe Lys Glu Gly
20 25 30

Ser Ala Leu Ser Ala Leu Asp Ala Ala Met Glu Lys Ile Ser Gly Lys
35 40 45

Pro Asp His Val Tyr Lys Asn Val Phe Ser Gly Phe Ala Ala Thr Leu
50 55 60

Asp Glu Asn Met Val Arg Val Leu Arg Ala His Pro Asp Val Glu Tyr
65 70 75 80

Ile Glu Gln Asp Ala Val Val Thr Ile Asn Ala Ala Gln Thr Asn Ala
85 90 95

Pro Trp Gly Leu Ala Arg Ile Ser Ser Thr Ser Pro Gly Thr Ser Thr
100 105 110

Tyr Tyr Tyr Asp Glu Ser Ala Gly Gln Gly Ser Cys Val Tyr Val Ile
115 120 125

Asp Thr Gly Ile Glu Ala Ser His Pro Glu Phe Glu Gly Arg Ala Gln
130 135 140

Met Val Lys Thr Tyr Tyr Tyr Ser Ser Arg Asp Gly Asn Gly His Gly
145 150 155 160

Thr His Cys Ala Gly Thr Val Gly Ser Arg Thr Tyr Gly Val Ala Lys
165 170 175

Lys Thr Gln Leu Phe Gly Val Lys Val Leu Asp Asp Asn Gly Ser Gly
180 185 190

```
Gln Tyr Ser Thr Ile Ile Ala Gly Met Asp Phe Val Ala Ser Asp Lys
        195                     200                 205

Asn Asn Arg Asn Cys Pro Lys Gly Val Val Ala Ser Leu Ser Leu Gly
    210                     215                 220

Gly Gly Tyr Ser Ser Ser Val Asn Ser Ala Ala Ala Arg Leu Gln Ser
225                     230                 235                 240

Ser Gly Val Met Val Ala Val Ala Ala Gly Asn Asn Asn Ala Asp Ala
                245                 250                 255

Arg Asn Tyr Ser Pro Ala Ser Glu Pro Ser Val Cys Thr Val Gly Ala
            260                 265                 270

Ser Asp Arg Tyr Asp Arg Arg Ser Ser Phe Ser Asn Tyr Gly Ser Val
        275                 280                 285

Leu Asp Ile Phe Gly Pro Gly Thr Ser Ile Leu Ser Thr Trp Ile Gly
    290                 295                 300

Gly Ser Thr Arg Ser Ile Ser Gly Thr Ser Met Ala Thr Pro His Val
305                 310                 315                 320

Ala Gly Leu Ala Ala Tyr Leu Met Thr Leu Gly Lys Thr Thr Ala Ala
                325                 330                 335

Ser Ala Cys Arg Tyr Ile Ala Asp Thr Ala Asn Lys Gly Asp Leu Ser
            340                 345                 350

Asn Ile Pro Phe Gly Thr Val Asn Leu Leu Ala Tyr Asn Asn Tyr Gln
        355                 360                 365

Ala
```

<210> 2
<211> 1124
<212> DNA
<213> Tritirachium album Limber

<400> 2

```
gaattcgctc ctgccgttga gcagcgctcc gaggctgctc ctctgatcga ggcccgcggc 60
gagatggttg ccaacaagta catcgtcaag ttcaaggagg gtagcgctct ttccgctctg 120
gatgctgcca tggagaagat ctctggcaag cccgaccacg tctacaagaa cgtcttcagc 180
ggtttcgctg cgaccctgga cgagaacatg gttcgggttc tccgcgccca ccccgatgtt 240
gagtacatcg agcaggatgc tgttgtcacc atcaacgctg cgcagaccaa cgctccctgg 300
ggcctggctc gcatctccag caccagcccc ggtacctcta cctactacta tgacgaatct 360
gccggccaag gctcctgcgt ctacgtgatc gacaccggta tcgaggcatc gcaccccgag 420
ttcgagggtc gtgcccagat ggtcaagacc tactactact ccagtcgcga cggtaacggt 480
cacggcaccc actgcgctgg taccgttggc tcccgtacct acggtgtcgc caagaagacc 540
cagctgttcg gtgtcaaggt cctggatgac aacggcagtg gccagtactc caccatcatc 600
gccggtatgg acttcgttgc cagcgacaag aacaaccgca actgccccaa aggtgtcgtt 660
gcctccttat ccctgggcgg tggttactcc tcctccgtga acagcgccgc tgcccgcctc 720
cagagctctg gtgtcatggt cgccgtcgct gccggtaaca acaacgctga cgcccgcaac 780
tactcccctg cttctgagcc ctcggtctgc accgtcggtg cttctgaccg ctacgaccgc 840
cgctccagct tctccaacta cggcagcgtt ttggacatct tcggccctgg taccagcatc 900
ctctccacct ggatcggcgg cagcacccgc tccatctctg gtacctccat ggctactccc 960

cacgttgccg gtctcgctgc ctacctcatg actcttggaa agactaccgc cgccagcgct 1020
tgccgataca ttgccgacac cgccaacaag ggcgacttaa gcaacattcc cttcggcact 1080
gtcaacttgc ttgcctacaa caactaccag gcttaatgaa gctt                 1124
```

<210> 3
<211> 79
<212> DNA
<213> Artificial Sequence

<220>
<223> Description of Artificial Sequence: Primer

<400> 3

```
cgcgaattcg ctcctgccgt tgagcagcgc tccgaggctg ctcctctgat cgaggcccgc 60
ggcgagatgg ttgccaaca                                              79
```

<210> 4
<211> 80
<212> DNA
<213> Artificial Sequence

<220>
<223> Description of Artificial Sequence: Primer

<400> 4

```
atcttctcca tggcagcatc cagagcggaa agagcgctac cctccttgaa cttgacgatg 60
tacttgttgg caaccatctc                                             80
```

<210> 5
<211> 80
<212> DNA
<213> Artificial Sequence

<220>
<223> Description of Artificial Sequence: Primer

<400> 5

```
tgccatggag aagatctctg gcaagcccga ccacgtctac aagaacgtct tcagcggttt 60
cgctgcgacc ctggacgaga                                             80
```

<210> 6
<211> 64
<212> DNA
<213> Artificial Sequence

<220>
<223> Description of Artificial Sequence: Primer

<400> 6

```
tgctcgatgt actcaacatc ggggtgggcg cggagaaccc gaaccatgtt ctcgtccagg 60
gtcg                                                              64
```

<210> 7
<211> 65
<212> DNA
<213> Artificial Sequence

<220>
<223> Description of Artificial Sequence: Primer

<400> 7

```
tgagtacatc gagcaggatg ctgttgtcac catcaacgct gcgcagaccg ctgcgcagac 60
caacg                                                             65
```

<210> 8
<211> 70
<212> DNA
<213> Artificial Sequence

<220>
<223> Description of Artificial Sequence: Primer

<400> 8

```
agtaggtaga ggtaccgggg ctggtgctgg agatgcgagc caggccccag ggagcgttgg 60
tctgcgcagc                                                        70
```

<210> 9
<211> 80
<212> DNA
<213> Artificial Sequence

<220>
<223> Description of Artificial Sequence: Primer

<400> 9

```
gtacctctac ctactactat gacgaatctg ccggccaagg ctcctgcgtc tacgtgatcg 60
acaccggtat cgaggcatcg                                             80
```

<210> 10
<211> 81
<212> DNA
<213> Artificial Sequence

<220>
<223> Description of Artificial Sequence: Primer

<400> 10

```
ttaccgtcgc gactggagta gtagtaggtc ttgaccatct gggcacgacc ctcgaactcg 60
gggtgcgatg cctcgatacc g                                           81
```

<210> 11
<211> 78
<212> DNA
<213> Artificial Sequence

<220>
<223> Description of Artificial Sequence: Primer

<400> 11

```
ccagtcgcga cggtaacggt cacggcaccc actgcgctgg taccgttggc tcccgtacct 60
acggtgtcgc caagaaga                                               78
```

<210> 12
<211> 73
<212> DNA
<213> Artificial Sequence

<220>
<223> Description of Artificial Sequence: Primer

<400> 12

```
atggtggagt actggccact gccgttgtca tccaggacct tgacaccgaa cagctgggtc 60
ttcttggcga cac                                                    73
```

<210> 13
<211> 81
<212> DNA
<213> Artificial Sequence

<220>
<223> Description of Artificial Sequence: Primer

<400> 13

ggccagtact ccaccatcat cgccggtatg gacttcgttg ccagcgacaa gaacaaccgc 60
aactgcccca aaggtgtcgt t 81

<210> 14
<211> 81
<212> DNA
<213> Artificial Sequence

<220>
<223> Description of Artificial Sequence: Primer

<400> 14

gctctggagg cgggcagcgg cgctgttcac ggaggaggag taaccaccgc ccagggataa 60
ggaggcaacg acacctttgg g 81

<210> 15
<211> 82
<212> DNA
<213> Artificial Sequence

<220>
<223> Description of Artificial Sequence: Primer

<400> 15

gcccgcctcc agagctctgg tgtcatggtc gccgtcgctg ccggtaacaa caacgctgac 60
gcccgcaact actcccctgc tt 82

<210> 16
<211> 80
<212> DNA
<213> Artificial Sequence

<220>
<223> Description of Artificial Sequence: Primer

<400> 16

gttggagaag ctggagcggc ggtcgtagcg gtcagaagca ccgacggtgc agaccgaggg 60
ctcagaagca ggggagtagt 80

<210> 17
<211> 83
<212> DNA
<213> Artificial Sequence

<220>
<223> Description of Artificial Sequence: Primer

<400> 17

ctccagcttc tccaactacg gcagcgtttt ggacatcttc ggccctggta ccagcatcct 60
ctccacctgg atcggcggca gca 83

<210> 18
<211> 81
<212> DNA
<213> Artificial Sequence

<220>
<223> Description of Artificial Sequence: Primer

<400> 18

tcatgaggta ggcagcgaga ccggcaacgt ggggagtagc catggaggta ccagagatgg 60
agcgggtgct gccgccgatc c 81

<210> 19
<211> 81
<212> DNA
<213> Artificial Sequence

<220>
<223> Description of Artificial Sequence: Primer

<400> 19

ctgcctacct catgacctta ggaaagacca ccgccgccag cgcttgccgt tacatcgccg 60
acaccgccaa caagggcgac t 81

<210> 20
<211> 87
<212> DNA
<213> Artificial Sequence

<220>
<223> Description of Artificial Sequence: Primer

<400> 20

atataagctt ctattaagcc tggtagttgt tgtaggctaa caggttgacg gtgccgaagg 60
gaatgttgct taagtcgccc ttgttgg 87

<210> 21
<211> 384
<212> PRT
<213> Tritirachium album Limber

<400> 21

```
Met Arg Leu Ser Val Leu Leu Ser Leu Leu Pro Leu Ala Leu Gly Ala
1               5                   10                  15

Pro Ala Val Glu Gln Arg Ser Glu Ala Ala Pro Leu Ile Glu Ala Arg
            20                  25                  30

Gly Glu Met Val Ala Asn Lys Tyr Ile Val Lys Phe Lys Glu Gly Ser
        35                  40                  45

Ala Leu Ser Ala Leu Asp Ala Ala Met Glu Lys Ile Ser Gly Lys Pro
    50                  55                  60
```

```
Asp His Val Tyr Lys Asn Val Phe Ser Gly Phe Ala Ala Thr Leu Asp
 65                 70                  75                  80

Glu Asn Met Val Arg Val Leu Arg Ala His Pro Asp Val Glu Tyr Ile
                85                  90                  95

Glu Gln Asp Ala Val Val Thr Ile Asn Ala Ala Gln Thr Asn Ala Pro
            100                 105                 110

Trp Gly Leu Ala Arg Ile Ser Ser Thr Ser Pro Gly Thr Ser Thr Tyr
        115                 120                 125

Tyr Tyr Asp Glu Ser Ala Gly Gln Gly Ser Cys Val Tyr Val Ile Asp
    130                 135                 140

Thr Gly Ile Glu Ala Ser His Pro Glu Phe Glu Gly Arg Ala Gln Met
145                 150                 155                 160

Val Lys Thr Tyr Tyr Tyr Ser Ser Arg Asp Gly Asn Gly His Gly Thr
                165                 170                 175

His Cys Ala Gly Thr Val Gly Ser Arg Thr Tyr Gly Val Ala Lys Lys
            180                 185                 190

Thr Gln Leu Phe Gly Val Lys Val Leu Asp Asp Asn Gly Ser Gly Gln
        195                 200                 205

Tyr Ser Thr Ile Ile Ala Gly Met Asp Phe Val Ala Ser Asp Lys Asn
    210                 215                 220

Asn Arg Asn Cys Pro Lys Gly Val Val Ala Ser Leu Ser Leu Gly Gly
225                 230                 235                 240

Gly Tyr Ser Ser Ser Val Asn Ser Ala Ala Ala Arg Leu Gln Ser Ser
                245                 250                 255

Gly Val Met Val Ala Val Ala Ala Gly Asn Asn Asn Ala Asp Ala Arg
            260                 265                 270

Asn Tyr Ser Pro Ala Ser Glu Pro Ser Val Cys Thr Val Gly Ala Ser
        275                 280                 285

Asp Arg Tyr Asp Arg Arg Ser Ser Phe Ser Asn Tyr Gly Ser Val Leu
    290                 295                 300

Asp Ile Phe Gly Pro Gly Thr Ser Ile Leu Ser Thr Trp Ile Gly Gly
305                 310                 315                 320

Ser Thr Arg Ser Ile Ser Gly Thr Ser Met Ala Thr Pro His Val Ala
                325                 330                 335

Gly Leu Ala Ala Tyr Leu Met Thr Leu Gly Lys Thr Thr Ala Ala Ser
            340                 345                 350

Ala Cys Arg Tyr Ile Ala Asp Thr Ala Asn Lys Gly Asp Leu Ser Asn
        355                 360                 365

Ile Pro Phe Gly Thr Val Asn Leu Leu Ala Tyr Asn Asn Tyr Gln Ala
    370                 375                 380
```

## Beschreibung

Die vorliegende Erfindung betrifft ein Verfahren zur Herstellung von rekombinanter Proteinase K in löslicher und aktiver Form in wirtschaftlich interessanten Mengen

Proteinase K (E.C. 3.4.21.64, bekannt auch als Endopeptidase K) ist eine extrazelluläre Endopeptidase, die von dem Pilz *Tritirachium album* Limber synthetisiert wird. Sie gehört zur Klasse der Serinproteasen mit der typischen katalytischen Triade $Asp^{39}$-$His^{69}$-$Ser^{224}$ (Jany, K.D. et al. (1986) *FEBS Letters* Vol. 199(2), 139-144). Da die Sequenz der 279 Aminosäuren langen Polypeptidkette (Gunkel, F.A. und Gassen, H.G. (1989) *Eur. J. Biochem.* Vol. 179(1), 185-194) und die dreidimensionale Struktur (Betzel, C. et al. (1988) *Eur. J. Biochem.* Vol. 178(1), 155-71) eine hohe Homologie mit den bakteriellen Subtilisinen aufweist, wird Proteinase K zur Subtilisinfamilie gezählt (Pahler, A. et al. (1984) *EMBO J.* Vol. 3(6), 1311-1314; Jany, K.D. und Mayer, B. (1985). *Biol. Chem. Hoppe-Seyler,* Vol. 366(5), 485-492). Benannt wurde die Proteinase K auf Grund ihrer Fähigkeit, natives Keratin zu hydrolysieren und somit dem Pilz ein Wachstum auf Keratin als einziger C- und N-Quelle zu ermöglichen (Ebeling, W. et al. (1974) *Eur. J. Biochem.* Vol. 47 (1), 91-97). Proteinase K ist mit einer spezifischen Aktivität von über 30 U/mg eine der aktivsten bekannten Endopeptidasen (Betzel, C. et al. (1986)/*FEBS Lett.* Vol. 197(1-2), 105-110) und hydrolysiert unspezifisch native sowie denaturierte Proteine.

Die Proteinase K aus *Tritirachium album* Limber wird im natürlichen Wirt als Präproprotein translatiert. 1989 wurde durch Gunkel, F.A. und Gassen, H.G. (1989) *Eur. J. Biochem.* Vol. 179(1), 185-194 die Sequenz der cDNA des Gens, das für die Proteinase K codiert, entschlüsselt. Demnach setzt sich das Gen für die Präproproteinase K aus 2 Exons zusammen und codiert für eine 15 Aminosäuren lange Signalsequenz, eine 90 Aminosäuren lange Prosequenz und eine 279 Aminosäuren lange reife Proteinase K. Ein 63 bp langes Intron befindet sich im Bereich der Prosequenz. Das Präpeptid wird bei der Translokation in das endoplasmatische Retikulum (ER) abgespalten. Über die daran anschließende Prozessierung zur reifen Proteinase K unter Abspaltung des Propeptids ist heute noch sehr wenig bekannt.

Die reife Proteinase K besteht folglich aus 279 Aminosäuren. Die kompakte Struktur wird durch zwei Disulfidbrücken und zwei gebundene Calciumionen stabilisiert. Dies erklärt, warum Proteinase K im Vergleich zu anderen Subtilisinen eine deutlich höhere Stabilität gegenüber extremen pH-Werten, hohen Temperaturen, chaotropen Substanzen und Detergenzien zeigt (Dolashka, P. et al. (1992) *Int. J. Pept. Protein. Res.* Vol. 40(5), 465-471). Proteinase K zeichnet sich durch eine hohe Thermostabilität (bis 65°C, Bajorath et al. (1988), *Eur. J. Biochem.* Vol. 176,441-447) und einen weiten pH-Bereich (pH 7,5-12,0, Ebeling, W. et al. (1974) *Eur. J. Biochem.* Vol. 47(1), 91-97) aus. Die Aktivität wird in Anwesenheit von denaturierenden Substanzen wie Harnstoff oder SDS gesteigert (Hilz, H. et al. (1975) *J. Biochem.* Vol. 56(1), 103-108; Jany, K.D. und Mayer, B. (1985) *Biol. Chem. Hoppe-Seyler,* Vol. 366(5),485-492).

Die oben genannten Eigenschaften machen die Proteinase K insbesondere bei solchen biotechnologischen Anwendungen interessant, bei denen ein unspezifischer Proteinabbau erforderlich ist. Besonders zu erwähnen ist hier die Nukleinsäureisolierung (DNA oder RNA) aus Rohextrakt und die Probenvorbereitung bei der DNA-Analytik (Goldenberger, D. et al. (1995) PCR Methods Appl. Vol. 4(6), 368-370; US 5,187,083; US 5,346,999). Weitere Anwendungen liegen im Bereich der Proteinanalytik wie z.B. der Strukturaufklärung.

Proteinase K wird kommerziell in großen Mengen durch Fermentation des Pilzes *Tritirachium album* Limber (z.B. CBS 348.55, Merck Stamm No. 2429 oder Stamm ATCC 22563) gewonnen. Die Produktion der Proteinase K wird dabei jedoch durch Glucose oder freie Aminosäuren supprimiert. Da proteinhaltige Medien darüber hinaus die Expression der Proteasen induzieren, müssen als einzige Stickstoffquelle Proteine wie BSA, Milchpulver oder Sojabohnenmehl verwendet werden. Die Sekretion der Protease beginnt, sobald die stationäre Phase des Wachstums erreicht ist (Ebeling, W. et al. (1974) *Eur. J. Biochem.* Vol. 47(1), 91-97).

Da demzufolge *Tritirachium album* Limber für eine Fermentation in großem Maßstab schlecht geeignet ist und darüber hinaus genetisch schwer manipulierbar ist, wurden verschiedene Versuche unternommen, eine Überexpression von rekombinanter Proteinase K in anderen Wirtszellen zu erreichen. Wegen mangelnder Expression, Bildung von inaktiven "inclusion bodies" oder Problemen bei der Naturierung konnten jedoch bei diesen Versuchen keine signifikante Aktivität nachgewiesen werden (Gunkel, F.A. und Gassen, H.G. (1989) *Eur. J. Biochem.* Vol. 179(1), 185-194; Samal, B.B. et al. (1996) *Adv. Exp. Med. Biol.* Vol. 379, 95-104).

Tritirachium album Limber ist ein langsam wachsender Pilz, der nur geringe Mengen an Proteasen in das Medium sezerniert. Nachteilig ist der im Vergleich zu Hefe langsamere Zellzyklus sowie die geringere erreichbare optische Dichte im Fermenter. Außerdem ist es bekannt, daß T. album außer Proteinase K auch andere Proteasen produziert, welche die Präparation verunreinigen könnten (Samal, B.B. et al. (1991). *Enzyme Microb. Technol.* Vol. 13, 66-70).

Die Expression der Proteinase K in E. coli ist zwar prinzipiell möglich, erfolgt jedoch nicht in löslicher Form, sondern in sogenannten "inclusion bodies", aus denen das Enzym anschließend durch bestimmte Maßnahmen solubilisiert und renaturiert werden muß. Der Nachteil dieser Methode ist, daß bei der Solubilisierung und Renaturierung viel Protein verloren geht.

Aufgabe der vorliegenden Erfindung ist es daher, ein Verfahren zur Herstellung von rekombinanter Proteinase K in wirtschaftlich interessanten Mengen zur Verfügung zu stellen.

Es hat sich überraschenderweise gezeigt, daß es möglich ist, eine rekombinante Proteinase K als zymogene Vorstufe in löslicher Form in Hefe zu exprimieren und zu sekretieren, wobei eine autokatalytische Aktivierung zur aktiven Proteinase K erfolgt. Ein weiterer Gegenstand der Erfindung ist die Aufreinigung der aktiven Proteinase K aus dem Mediumüberstand.

Gegenstand der vorliegenden Erfindung ist daher ein Verfahren zur Herstellung von rekombinanter Proteinase K umfassend die Schritte

a) Transformation einer Wirtszelle mit einem Vektor enthaltend eine für die zymogene Vorstufe der Proteinase K kodierende DNA, wobei diese stromaufwärts der kodierenden Sequenz mit einer Sequenz im Leserahmen fusioniert ist, welche für ein Signalpeptid kodiert und unter Kontrolle eines für die Wirtszelle geeigneten Promotors steht,
b) Expression der zymogenen Vorstufe der Proteinase K
c) Sekretion und autokatalytische Aktivierung der Proteinase K
d) Isolierung und Reinigung der Proteinase K,

dadurch gekennzeichnet, daß es sich bei der Wirtszelle um Hefezellen ausgewählt aus der Gruppe bestehend aus Pichia spezies, Hansenula spezies, Saccharomyces spezies, und Schizosaccharomyces spezies handelt und das Protein in löslicher Form von diesem Expressionswirt sekretiert wird.

Besonders bevorzugt ist erfindungsgemäß, wenn *Pichia pastoris* als Wirtszelle verwendet wird.

Als vorteilhaft hat sich für das erfindungsgemäße Verfahren ferner herausgestellt, wenn die Wirtszelle mit einer für die zymogene Vorstufe kodierende DNA transformiert wird und die Proteinase K zu einem späteren Zeitpunkt während oder unmittelbar nach der Sekretion in das Kulturmedium autokatalytisch aktiviert wird.

Bei Verwendung von Pichia pastoris als Wirtszelle, wird das für die zymogene Vorstufe der Proteinase K kodierende Gen bevorzugt in folgende Vektoren kloniert: pPICZ, pPICZα, pGAPZ, pGAPZα, pPICZαA und pPIC9K . Besonders bevorzugt sind in diesem Fall die Vektoren: pPICZαA und pPIC9K. Insbesondere bevorzugt ist erfindungsgemäß der Vektor pPICZαA. Die vorstehend genannten Vektoren sind kommerziell erhältlich (Invitrogen).

Weiterhin ist gemäß dem erfindungsgemäßen Verfahren zur Herstellung von rekombinanter Proteinase K bevorzugt, wenn die Expression der Proteinase K bzw. deren zymogene Vorstufe der Proteinase K durch Methanol (pPIC-Vektoren) induziert wird. Eine andere Möglichkeiten ist die Induzierung der Expression durch Glycerinaldehydphosphat (pGAP-Vektoren).

Bei dem erfindungsgemäßen Verfahren zur Herstellung von rekombinanter Proteinase K wird die Sekretion des Proteins bevorzugt durch die N-terminale Fusionierung des Signalpeptides des α-Faktors aus Saccharomyces cerevisiae eingeleitet. Das bedeutet beispielsweise bei den oben genannten Vektoren, die mit α gekennzeichnet sind, daß diese die Nukleotidsequenz für das Signalpeptid des α-Faktors aus Saccharomyces cerevisiae besitzen. Bei der Translation wird dann ein Fusions-Protein aus Signalpeptid am N-Terminus und Zielprotein erzeugt. Ein weiteres mögliches Signalpeptid wäre die natürliche Signalsequenz der Proteinase K.

Als besonders bevorzugt hat sich ferner erwiesen, wenn zur Herstellung der rekombinanten Proteinase K die Wirtszelle Pichia pastoris mit dem Expressionsvektoren pPICZαA und pPIC9K, welche eine für die zymogene Vorstufe kodierende DNA enthalten, transformiert wird und das Gen unter Kontrolle des AOX1-Promotors und gegebenenfalls des AOX1-Terminators steht.

Gegenstand der vorliegenden Erfindung ist ebenfalls ein Vektor enthaltend eine für die zymogene Vorstufe der Proteinase K kodierende DNA, wobei diese stromaufwärts der kodierenden Sequenz mit einer Sequenz im Leserahmen fusioniert ist, welche für ein geeignetes Signalpeptid kodiert und wobei das kodierende Gen unter Kontrolle eines für die Wirtszelle geeigneten Promotors und gegebenenfalls Terminators steht und wobei dieser Vektor für die Transformation dieser Wirtszelle geeignet ist. Erfindungsgemäß ist die Wirtszelle eine Hefe ausgewählt aus der Gruppe bestehend aus Pichia species, Hansenula species, Saccharomyces species und Schizosaccharomyces species.

Gegenstand der Erfindung ist somit ferner ein rekombinanter Vektor, der eine oder mehrere Kopien der vorstehend definierten rekombinanten DNA enthält. Der Vektor ist bevorzugt ein Plasmid, welches einen für die Wirtszelle starken Promotor und ein für die Wirtszelle geeignetes Signalpeptid zur Sekretion von Proteinen besitzt. Denkbar ist aber darüber hinaus die Fusion des nativen Signalpeptides der Präproproteinase K an den N-Terminus des Propeptides, wie es in SEQ. ID. NO.: 21 (Signalsequenz 1-15 (15 Aminosäuren); Prosequenz 16-104 (90 Aminosäuren); Sequenz der reifen Proteinase K 106-384 (279 Aminosäuren)) dargestellt ist. Zur Herstellung des Expressionsvektors werden Methoden verwendet, die dem Fachmann geläufig sind und zum Beispiel bei Sambrook et al. (1989) beschrieben sind.

Ein weiterer Gegenstand der vorliegenden Erfindung ist eine Wirtszelle transformiert mit einem der oben aufgeführten Vektoren, wobei die Wirtszelle eine Hefe ist, ausgewählt aus der Gruppe bestehend aus. Pichia spezies, Hansenula spezies wie z.B. Hansenula polymorpha, Saccharomyces spezies, und Schizosaccharomyces Spezies. Besonders bevorzugt ist als Wirtszelle Pichia pastoris. Insbesondere ist bevorzugt, wenn mehrere Vektoren(mit jeweils einer Kopie des *ppK*-Gens) in das Genom integriert werden.

Für die Aufreinigung der Proteinase K werden in einem ersten Schritt die Hefezellen über Mikrofiltration bzw. Zentrifugation entfernt. Die resultierende klare Lösung enthält die Protease. Anschließend folgt eine Umpufferung über Ultrafiltration, um das Produkt an einen Kationenaustauscher, bei-spielsweise SP-Sepharose oder SP-Sephadex (Pharmacia) oder SP-Toyopearl (Tosoh Corporation) zu binden. Nach der Elution erfolgt eine erneute Umpufferung über Ultrafiltration und die Bindung an einen Anionenaustauscher, beispielsweise DEAE-Sepharose oder Q-Sepharose (Pharmacia) oder DEAE-Fraktogel (Merck). Nach der erneuten Elution wird die reine Protease über Ultrafiltration in ein stabiles Puffersystem überführt (Protein Purification, Principles and Practice, Robert K. Scopes, Springer Verlag, 1982). Der Fachmann kann jedoch auch andere Methoden zur Reinigung verwenden, die zum Stand der Technik gehören.

Durch das erfindungsgemäße Verfahren ist es überraschenderweise gelungen, rekombinante Proteinase K herzustellen, wobei das Enzym löslich und aktiv von einer heterologen Wirtszelle produziert wird. Bei der Expression der Proteinase K mit anschließender Sekretion des Enzyms in das Kulturmedium ist insbesondere von Vorteil, daß verhindert wird, daß die Proteinase K im Cytosol der Wirtszelle eine stark toxische Wirkung entfaltet. Weiterhin wird die korrekte Ausbildung der zwei Disulfidbrücken gewährleistet, die im reduzierenden Milieu des Cytosols auch nicht ohne weiteres stattfinden könnte. Somit ist ein entscheidender Vorteil des erfindungsgemäßen Verfahrens, daß ein Weg für die lösliche und aktive Produktion einer rekombinanten Proteinase K bereitgestellt wird. Es ist sehr überraschend und nicht erklärlich, daß die Oberflächenproteine der erfindungsgemäßen Wirtszellen durch eine sekretierte Proteinase K nicht hydrolysiert werden. Bei der zu erwartenden Hydrolyse der Oberflächenproteine durch Proteinase K würde die Wirtszelle in ihrem Lebenszyklus gestört.

Durch das erfindungsgemäße Verfahren wird eine Proteinase K erhalten, welche homogen ist und daher in besonderem Maße für analytische und diagnostische Anwendungen geeignet ist. Die erfindungsgemäße zymogene Vorstufe der Proteinase K kann gegebenenfalls weitere N-terminale Modifikationen enthalten, und zwar insbesondere Sequenzen, die eine Reinigung des Zielproteins erleichtern ("affinity tags"). Ferner kann die zymogene Vorstufe Sequenzen enthalten, welche die Effizienz der Translation erhöhen, welche die Faltungseffizienz steigern und/oder auch solche Sequenzen, die zu einer Sekretion des Zielproteins in das Kulturmedium führen (natürliche Präsequenz und andere Signalpeptide).

Unter Proteinase K im Sinne der Erfindung sind bevorzugt sowohl die in SEQ. ID. NO.: 1 angegebene Sequenz nach Gassen et al. (1989), als auch andere Varianten der Proteinase K aus Tritirachium album Limber, wie die in Ch. Betzel et al. (Biochemistry 40 (2001), 3080-3088) offenbarte Aminosäuresequenz sowie insbesondere Proteinase T (Samal, B.B. et al. (1989) Gene Vol. 85(2), 329-333; Samal, B.B. et al. (1996) Adv. Exp. Med. Biol. Vol. 379, 95-104) und Proteinase R (Samal, B.B. et al. (1990) Mol. Microbiol. Vol. 4(10), 1789-1792; US 5,278,062) und darüber hinaus mit rekombinanten Mitteln erzeugte Varianten (wie zum Beispiel in der WO 96/28556 beschrieben) zu verstehen. SEQ. ID. NO.: 1 umfaßt eine Prosequenz (1-90; 90 Aminosäuren) und die Sequenz der reifen Proteinase K (91-368; 279 Aminosäuren). Die von Betzel et al. (Biochemistry 40 (2001), 3080-3088) beschriebene Proteinase-K-Aminosäuresequenz weist insbesondere in Position 207 der aktiven Protease Aspartat anstatt eines Serinrestes auf.

Pro-Proteinase K im Sinne der Erfindung bedeutet insbesondere eine Proteinase K, die am N-Terminus mit ihrer Prosequenz gemäß SEQ. ID. NO.: 1 verknüpft ist. Bei dem mit Proteinase K eng verwandten Subtilisin E und entsprechenden Varianten hat die Prosequenz einen essentiellen Einfluß auf die Faltung und Bildung von aktiver Protease (Ikemura, H. et al. (1987) Biol. Chem. Vol. 262(16), 7859-7864). Es wird insbesondere vermutet, daß die Prosequenz als intramolekulares Chaperon wirkt (Inouye, M. (1991) Enzyme Vol. 45, 314-321). Nach der Faltung findet die Prozessierung zur reifen Subtilisin-Protease statt, indem das Propeptid autokatalytisch abgespalten wird (Ikemura, H. und Inouye, M. (1988) J. Biol. Chem. Vol. 263(26), 12959-12963). Dieser Prozeß findet bei Subtilisin E (Samal, B.B. et al. (1989) Gene Vol. 85(2), 329-333; Volkov, A. und Jordan, F. (1996) J. Mol. Biol. Vol. 262, 595-599), Subtilisin BPN' (Eder, J. et al. (1993) Biochemistry Vol. 32, 18-26), Papain (Vernet, T. et al. (1991) J. Biol. Chem. Vol. 266(32), 21451-21457) und Thermolysin (Marie-Claire, C. (1998) J. Biol. Chem. Vol. 273(10), 5697-5701) intramolekular statt.

Für die Funktion als Chaperon scheinen nur bestimmte, meist hydrophobe Kernbereiche der Prosequenz notwendig zu sein, da Mutationen in weiten Bereichen ohne Einfluß auf die Aktivität bleiben (Kobayashi, T. und Inouye, M. (1992) J. Mol. Biol. Vol. 226, 931-933). Außerdem ist bekannt, daß Propeptide zwischen verschiedenen Subtilisinvarianten austauschbar sind. So erkennt zum Beispiel Subtilisin BPN' auch die Prosequenz von Subtilisin E (Hu, Z. et al. (1996) J. Biol. Chem. Vol. 271 (7), 3375-3384).

Bevorzugt ist somit sowohl die 90 Aminosäuren lange Prosequenz nach SEQ. ID. NO.: 1, als auch andere Varianten, die eine faltungsfördernde Funktion erfüllen. Ebenfalls bevorzugt ist ein Propeptid, das exogen zu der Faltung von reifer Proteinase K gegeben wird und die oben ausgeführten Funktionen erfüllt.

Der wesentliche Vorteil des erfindungsgemäßen Verfahrens ist daher, daß die rekombinante Proteinase K löslich und aktiv von einem Expressionswirt in das Kulturmedium sekretiert wird. Darüber hinaus wird der bei dem erfindungsgemäßen Verfahren eingesetzte Expressionswirt durch die sehr aktive und unspezifische Protease nicht geschädigt oder in sonstiger Weise negativ beeinflußt, d.h. insbesondere, daß das Wachstum weiter problemlos erfolgt und eine verstärkte Zell-Lyse nicht zu beobachten ist. Zudem ist der erfindungsgemäße Expressionswirt im Vergleich zu Tritirachium album leichter handhabbar und zeichnet sich durch höhere Wachstumsraten aus.

**Figurenbeschreibung**

Figur 1
Expressionsplasmid pPICPK-1. Eine für die zymogene Proform der Proteinase K kodierende Sequenz kloniert in den Ausgangsvektor pPICZαA (Invitrogen).

Figur 2
Expressionsplasmid pPICPK-2. Eine für die zymogene Proform der Proteinase K kodierende Sequenz kloniert in den Ausgangsvektor pPIC9K (Invitrogen).

**Beispiele**

Beispiel 1

*Gensynthese*

Das Gen für die reife Proteinase K aus Tritirachium album Limber ohne Signalsequenz und ohne Intron wurde mittels Gensynthese generiert. Als Vorlage wurde die 368 Aminosäuren lange Sequenz (ohne natives Signalpeptid) von Gunkel und Gassen, 1989 verwendet. Durch Rücktranslation der Aminosäuresequenz wurde eine für die Expression sowohl in E.coli wie auch Hefe codon-optimierte Nukleinsäuresequenz erstellt. Die Aminosäuresequenz ist in SEQ. ID. NO.: 1, die Nukleotidsequenz in SEQ. ID. NO.: 2 dargestellt.
Für die Gensynthese wurde das Gen in 18 Fragmente aus Sinn- und reversen, komplementären Gegenstrangoligonukleotiden in alternierender Folge unterteilt (SEQ. ID. NO.: 3-20). Am 5'- und 3'-Ende wurde jeweils ein mindestens 15 bp- langer Bereich angehängt, der jeweils mit den benachbarten Oligonukleotiden überlappt. An die 5'- und 3'-Enden des synthetischen Gens wurden außerhalb des codierenden Bereichs zur späteren Klonierung in Expressionsvektoren Erkennungsstellen für Restriktionsendonukleasen angehängt. Für die Klonierung des pro-Proteinase K-Gens wurde als 5'-Primer das in SEQ. ID. NO.: 3 dargestellte Oligonukleotid verwendet, das eine EcoRI-Schnittstelle enthält. SEQ. ID. NO.: 20 zeigt den 3'-Primer mit HindIII-Schnittstelle. Der 3'-Primer enthält ein zusätzliches Stoppcodon nach dem natürlichen Stoppcodon, um eine gesicherte Termination der Translation zu gewährleisten.
Die Oligonukleotide wurden mittels PCR-Reaktion miteinander verknüpft und das daraus resultierende Gen amplifiziert. Dabei wurde das Gen zunächst in drei Fragmente zu je 6 Oligonukleotiden unterteilt und in einem zweiten PCR-Zyklus die drei Teilstücke miteinander verknüpft.
Fragment 1 setzt sich aus den Oligonukleotiden wie in SEQ. ID. NO.: 3-8 dargestellt, Fragment 2 aus Oligonukleotide wie in SEQ. ID. NO.: 9-14 dargestellt und Fragment drei aus Oligonukleotid wie in SEQ. ID. NO.: 15-20 dargestellt, zusammen.

Folgende PCR-Parameter wurden angewandt:

PCR-Reaktion 1 (Generierung der drei Teilstücke)

| | | |
|---|---|---|
| 5 min | 95°C | hotstart |
| 2 min | 95°C | 30 Zyklen |
| 2 min | 42°C | |
| 1,5 min | 72°C | |
| 7 min | 72°C | final extension |

PCR-Reaktion 2 (Verknüpfung der Teilstücke zum Gesamtgen)

| | | | |
|---|---|---|---|
| 5 min | 95°C | | hotstart |
| 1,5 min | 95°C | } | 6 Zyklen (ohne endständige Primer) |
| 2 min | 48°C | | |
| 2 min | 72°C | | |

Zugabe der enständigen Primer

| | | | |
|---|---|---|---|
| 1,5 min | 95°C | } | 25 Zyklen (mit endständigen Primern) |
| 1,5 min | 60°C | | |
| 2 min | 72°C | | |
| 7 min | 72°C | | final extension |

Beispiel 2

*Klonierung des synthetischen Proteinase K-Fragmentes aus der Gensynthese*

Der PCR-Ansatz wurden auf ein Agarosegel aufgetragen und das ca. 1130 Bp große PCR-Fragment wurde aus dem Agarosegel (Geneclean II Kit von Bio 101, Inc. CA USA) isoliert. Das Fragment wurde mit den EcoRI- und HindIII-Restriktionsendonukleasen (Roche Diagnostics GmbH, Germany) 1 Stunde bei 37°C geschnitten. Gleichzeitig wurde das pUC18-Plasmid (Roche Diagnostocs GmbH, Germany) mit den EcoRI- und HindIII-Restriktionsendonukleasen 1 Stunde bei 37°C geschnitten, der Ansatz mittels Agarosegelelektorphorese aufgetrennt und das 2635 Bp große Vektorfragment isoliert. Anschließend wurden das PCR-Fragment und das Vektorfragment mittels T4-DNA-Ligase miteinander ligiert. Dazu wurden 1 µl (20 ng) Vektorfragment und 3 µl (100 ng) PCR-Fragment, 1µ l 10 x Ligase-Puffer (Maniatis et al., 1989 B.27), 1 µl T4-DNA-Ligase, 4 µl steriles $H_2O$ bidest pipettiert, vorsichtig gemischt und über Nacht bei 16°C inkubiert.

Das klonierte Gen wurde mittels Restriktionsanalyse und durch mehrfache Sequenzierung beider Stränge untersucht.

Beispiel 3

*Vektorkonstruktion*

Das synthetische Gen mußte zunächst wieder aus dem pUC-Plasmid isoliert werden. Zu diesem Zweck wurden 1 µg Plasmid-DNA zunächst mit der Restriktionsendonuklease HindIII (Roche Diagnostics GmbH) nach den Herstellerangaben inkubiert und anschließend die Restriktionsendonuklease durch Erwärmung auf 65°C für 20 min inaktiviert. Anschließend wurden dabei entstehenden DNA-Überhänge mit Klenow-Polymerase nach Herstellerangaben (Roche Diagnostics GmbH) aufgefüllt und die Klenow-Polymerase dann durch Inkubation bei 75°C für 10 min inaktiviert. Zuletzt wurde das nun linearisierte Vektorfragment des o.a. pUC-Plasmides mit der Restriktionsendonuklease EcoRI (Roche Diagnostics GmbH) nach den Herstellerangaben geschnitten, der Restriktionsansatz auf ein 1%iges Agarosegel aufgetragen und die Fragmente der Größe nach durch Anlegen von Strom (100 V/ 150 mA) voneinander getrennt. Das ca. 1120 bp große Fragment, enthaltend das Gen für die Proproteinase K (*ppk*-Gen) wurde aus dem Agarosegel isoliert (QIAquick Gel Extraction Kit/Qiagen).

Der Vektor pPICZαA (Invitrogen) wurde mit der Restriktionsendonuklease Asp718I (Roche Diagnostics GmbH) nach den Herstellerangaben geschnitten und Restriktionsendonuklease durch Erwärmung des Inkubationsansatzes auf 65°C für 20 min inaktiviert. Anschließend wurden dabei entstehenden DNA-Uberhänge mit Klenow-Polymerase nach Herstellerangaben (Roche Diagnostics GmbH) aufgefüllt und die Klenow-Polymerase dann durch Inkubation bei 75°C für 10 min inaktiviert. Zuletzt wurde das nun linearisierte Vektorfragment von pPICZαA mit der Restriktionsendonuklease EcoRI (Roche Diagnostics GmbH) nach den Herstellerangaben geschnitten, der Restriktionsansatz auf ein 1%iges Agarosegel aufgetragen und die Fragmente der Größe nach durch Anlegen von Strom (100 V/ 150 mA) voneinander getrennt. Das ca. 3550 bp große Vektorfragment wurde aus dem Agarosegel isoliert (QIAquick Gel Extraction Kit/Qiagen).

Die so gewonnenen Fragmente wurden nach Standardmethode (Sambrook et al. 1989) miteinander ligiert. In diesem Vektor steht das *ppk*-Gen unter Kontrolle des AOX 1-Promotors (Promotor für die Alkoholoxidase 1 aus Pichia pastoris, mit Methanol induzierbar) und wird mit dieser Klonierungsstrategie im korrekten Leserahmen hinter das Signalpeptid des α-Faktors aus Saccharomyces cerevisiae kloniert. Das so insertierte Genfragment wurde dann mittels Restriktionsanalyse und Sequenzierung auf fehlerfreie Basensequenz untersucht. Der so entstandene Expressionsvektor, der das *ppk*-Gen, das für die Proproteinase K kodiert, enthält, wurde pPICPK-1(s. Fig. 1) genannt.

Anschließend wurde das *ppk*-Gen ebenfalls in pPIC9K (Invitrogen) kloniert. Dazu wurde der Vektor pPICPK-1 mit den Restriktionsendonukleasen PmeI und NotI (Roche Diagnostics GmbH) nach den Herstellerangaben geschnitten, die Fragmente aus dem Restriktionsansatz in einem 1%igen Agarosegel der Größe nach aufgetrennt und das ca. 1960 bp große Fragment, enthaltend den 3'-Teil der AOX1-Promotor-Region, die Sequenz für das Signalpeptid des α-Faktors und das *ppk*-Gen, aus dem Gel isoliert (QIAquick Gel Extraction Kit/Qiagen). Gleichzeitig wurde der Vektor pPIC9K mit den Restriktionsendonukleasen PmeI und NotI (Roche Diagnostics GmbH) nach den Herstellerangaben geschnitten, die Fragmente aus dem Restriktionsansatz in einem 1%igen Agarosegel der Größe nach aufgetrennt und das ca. 8450 bp große Vektorfragment aus dem Gel isoliert (QIAquick Gel Extraction Kit/Qiagen).

Anschließend wurden die so gewonnenen Fragmente nach Standardmethode (Sambrook et al. 1989) miteinander ligiert. In diesem Vektor steht das *ppk*-Gen ebenfalls unter Kontrolle des AOX 1-Promotors (Promotor für die Alkoholoxidase 1 aus Pichia pastoris, mit Methanol induzierbar). Der Vektor pPIC9K unterscheidet sich von dem Vektor pPICZαA durch den Selektionsmarker und durch drei dem Fachmann bekannten Integrationsmöglichkeit in das *Pichia* Genom je nach Vektorlinearisierung vor der Transformation während die Integration von pICZαA in den AOX1-Locus festgelegt ist. Das insertierte Genfragment wurde dann mittels Restriktionsanalyse und Sequenzierung auf fehlerfreie Basensequenz untersucht.

Der so entstandene Expressionsvektor, der das *ppk*-Gen, das für die Proproteinase K kodiert, enthält, wurde pPICPK-2 (s. Fig. 2) genannt.

Beispiel 4

*Transformation von pPICPK-1 in Pichia pastoris*

Zur Transformation von pPICPK-1 in Pichia pastoris X-33 mit anschließender Integration in das Genom wurde der Vektor zunächst mit PmeI (Roche Diagnostics GmbH) linearisiert. Die Transformation wurde mittels Elektroporation mit dem Gene Pulser II (Biorad) durchgeführt.

Dazu wurde eine Kolonie von Pichia pastoris Wildtypstamm in 5 ml YPD-Medium (nach Invitrogen-Katalog) angeimpft und bei 30°C über Nacht unter Schütteln inkubiert. Die Übernachtkultur wurde anschließend 1:2000 in 200 ml frisches YPD-Medium (nach Invitrogen-Katalog) überimpft und über Nacht bei 30°C unter Schütteln inkubiert, bis eine $OD_{600}$ von 1,3 - 1,5 erreicht war. Die Zellen wurden abzentrifugiert (1500 x g / 5 Minuten) und das Pellet in 200 ml eiskaltem, sterilen Wasser (0°C) resuspendiert. Die Zellen wurden wiederum abzentrifugiert (1500 x g/5 Minuten) und in 100 ml eiskaltem, sterilen Wasser (0°C) resuspendiert. Die Zellen wurden wiederum abzentrifugiert, in 10 ml eiskaltem (0°C) 1 M Sorbitol (ICN) resuspendiert. Die Zellen wurden wiederum abzentrifugiert, in 0,5 ml eiskaltem (0°C) 1 M Sorbitol (ICN) resuspendiert. Die so gewonnenen Zellen wurden auf Eis gehalten und sofort zur Transformation eingesetzt.

80 µl der Zellen wurden mit ca. 1 µg linearisierter pPICPK-1-Vektor-DNA versetzt und der gesamte Ansatz in eine eiskalte (0°C) Elektroporationsküvette transferiert und weitere 5 Minuten auf Eis inkubiert. Anschließend wurde die Küvette in den Gene Pulser II (Biorad) überführt und die Transformation bei 1 kV, 1 kΩ und 25µF durchgeführt. Nach der Elektroporation wurde der Ansatz mit 1 ml 1M Sorbitol (ICN) versetzt und anschließend 100 - 150 µl auf eine YPDS-Agarplatte (nach Invitrogen-Katalog) mit 100µg/ml Zeocin® (Invitrogen) ausplattiert. Die Platten wurden anschließend 2 - 4 Tage bei 30 °C inkubiert.

Die Klone wurden auf Raster-MD (= minimal Dextrose)-Platten überimpft und weiter analysiert.

Gewachsene Klone wurden gepickt, in 20 µl steriles Wasser resuspendiert, mit 17,5 U Lyticase (Roche Diagnostics GmbH) aufgeschlossen (1 Stunde, 37°C) und direkt mittels PCR auf die korrekte Integration der *ppk*-Expressionskassette untersucht.

Klone, die die komplette Expressionskassette bei der Transformation in das Genom integriert haben, wurden dann in Expressionsversuchen eingesetzt.

Beispiel 5

*Transformation von pPICPK-2 in Pichia pastoris*

Zur Transformation von pICPK-2 in Pichia pastoris GS115 mit anschließender Integration in das Genom wurde der Vektor zunächst für Variante 1 mit PmeI (Roche Diagnostics GmbH) zur Integration in den AOXI-Locus und für

Variante II mit SalI (Roche Diagnostics GmbH) zur Integration in den His4-Locus linearisiert. Die Transformation wurde mittels Elektroporation mit dem Gene Pulser II (Biorad) durchgeführt.

Dazu wurde eine Kolonie von Pichia pastoris GS115 Wildtypstamm in 5 ml YPD-Medium (nach Invitrogen-Katalog) angeimpft und bei 30°C über Nacht unter Schütteln inkubiert. Die Übernachtkultur wurde anschließend 1:2000 in 200 ml frisches YPD-Medium (nach Invitrogen-Katalog) überimpft und über Nacht bei 30°C unter Schütteln inkubiert, bis eine $OD_{600}$ von 1,3 -1,5 erreicht war. Die Zellen wurden abzentrifugiert (1500 x g / 5 Minuten) und das Pellet in 200 ml eiskaltem, sterilen Wasser (0°C) resuspendiert. Die Zellen wurden wiederum abzentrifugiert (1500 x g/5 Minuten) und in 100 ml eiskaltem, sterilen Wasser (0°C) resuspendiert. Die Zellen wurden wiederum abzentrifugiert, in 10 ml eiskaltem (0°C) 1 M Sorbitol (ICN) resuspendiert. Die Zellen wurden wiederum abzentrifugiert, in 0,5 ml eiskaltem (0°C) 1 M Sorbitol (ICN) resuspendiert. Die so gewonnenen Zellen wurden auf Eis gehalten und sofort zur Transformation eingesetzt.

80 µl der Zellen wurden mit ca. 1 µg linearisierter pICPK-2-Vektor-DNA versetzt und der gesamte Ansatz in eine eiskalte (0°C) Elektroporationsküvette transferiert und weitere 5 Minuten auf Eis inkubiert. Anschließend wurde die Küvette in den Gene Pulser II (Biorad) überführt und die Transformation bei 1 kV, 1 kΩ und 25µF durchgeführt. Nach der Elektroporation wurde der Ansatz mit 1 ml IM Sorbitol (ICN) versetzt und anschließend 100 -150 µl auf eine MM-Agarplatte (Minimalmedium nach Invitrogen-Katalog) ohne Histidin ausplattiert. Die Platten wurden anschließend 2 - 4 Tage bei 30 °C inkubiert. Klone von Pichia pastoris GS115, die durch Mutation ein defektes His4-Gen (Histidinoldehydrogenase) besitzen, können auf diesen Platten nur wachsen, wenn Sie den Vektor pPICPK-2 integriert haben, der das funktionsfähige His4-Gen als Insert besitzt und somit die Defizienz in der Histidinbiosynthese aufhebt.

Die Klone wurden auf Raster-MD (= minimal Dextrose)-Platten überimpft und weiter analysiert. Gewachsene Klone wurden gepickt, in 20 µl steriles Wasser resuspendiert, mit 17,5 U Lyticase (Roche Diagnostics GmbH) aufgeschlossen (1 Stunde, 37°C) und direkt mittels PCR auf die korrekte Integration der *ppk*-Expressionskassette untersucht.

Klone, die die komplette Expressionskassette bei der Transformation in das Genom integriert haben, wurden dann in Expressionsversuchen eingesetzt.

Beispiel 6

*Expression der Proteinase K*

Positive Klone wurden in 10 ml BMGY-Medium (nach Invitrogen-Katalog) angeimpft und über Nacht bei 30°C unter Schütteln inkubiert. Anschließend wurden die OD bei 600 nm bestimmt und so in 10ml BMMY-Medium (nach Invitrogen-Katalog) überimpft, daß eine $OD_{600}$ von 1 resultierte. Das BMMY-Medium (nach Invitrogen-Katalog) enthält Methanol (Mallinckrodt Baker B.V.), das die Expression der Proteinase K über den AOX 1-Promotor induziert.

Die Schüttelkolben wurden bei 30°C unter Schütteln inkubiert, alle 24 Stunden Proben gezogen, die $OD_{600}$ bestimmt, ein Aktivitätstest auf Expression der Proteinase K durchgeführt und jeweils mit 0,5% Methanol (Mallinckrodt Baker B.V.) zur weiteren Induktion nachgefüttert. Die Expressionsversuche liefen über 168 Stunden.

Beispiel 7

*Aktivitätstest der sekretierten rekombinanten Proteinase K*

Für den Aktivitätstest der rekombinanten Proteinase K benötigt man $CaCl_2$ x $2H_2O$ (Merck ID-Nr. 102382), DMSO (Merck ID-Nr. 109678), das Substrat Suc-Ala-Ala-Pro-Phe-pNA (Roche Diagnostics ID-Nr. 0716766) und Tris-Base (Roche Diagnostics ID-Nr. 0153265).

Die Lösungen setzen sich wie folgt zusammen:

| | |
|---|---|
| Lösung 1: | 50 mmol/l Tris-Base; 10 mmol/L $CaCl_2$pH 8,2 |
| Lösung 2: | 125 mg Suc-Ala-Ala-Pro-Phe-pNA in 1 ml DMSO gelöst |

Die Zellen werden abzentrifugiert (5 min 10000 rpm Eppendorf-Tischzentrifuge) und der Überstand 1:500 in Lösung 1 verdünnt.

2 ml von Lösung 1 werden in eine Küvette pipettiert und mit 0,02 ml von Lösung 2 versetzt. Die beiden Lösungen werden vermischt und auf die Reaktionstemperatur von 25°C temperiert. Die Reaktion wird durch Zugabe von 0,05 ml des wie o.a. verdünnten Überstandes und nochmaligen Mischen gestartet, die Absorbtionsänderung bei 405 nm gemessen und das ΔA/min aus dem linearen Bereich gemessen. Die Auswertung erfolgt dann nach folgender Formel:

$$\text{Aktivität} = \frac{2{,}07}{\varepsilon \times 1 \times 0{,}05} \quad \Delta A/\text{min [U/ml Probe-Lösung]}$$

2,07 = Probevolumen
$\varepsilon_{405}$ = 10,4 [$mmol^{-1}$ x 1 x $cm^{-1}$]
1 = Schichtdicke der Küvette
0,05 = Volumen der zugegebenen Probe

## Patentansprüche

1. Verfahren zur Herstellung von rekombinanter Proteinase K umfassend die Schritte

   a) Transformation einer Wirtszelle mit einem Vektor enthaltend eine für die zymogene Vorstufe der Proteinase K kodierende DNA, wobei diese stromaufwärts der kodierenden Sequenz mit einer Sequenz im Leserahmen fusioniert ist, welche für ein Signalpeptid kodiert und unter Kontrolle eines für die Wirtszelle geeigneten Promotors steht,
   b) Expression der zymogenen Vorstufe der Proteinase K,
   c) Sekretion und autokatalytische Aktivierung der Proteinase K,

   **dadurch gekennzeichnet, daß** es sich bei der Wirtszelle um Hefezellen ausgewählt aus der Gruppe bestehend aus Pichia sp., Hansenula sp., Saccharomyces sp. und Schizosaccharomyces sp., handelt und das Protein löslich von diesem Expressionswirt sekretiert wird.

2. Verfahren gemäß einem der Ansprüche 1 oder 2, wobei es sich bei der Wirtszelle um Pichia pastoris handelt.

3. Verfahren gemäß einem der Ansprüche 1 und 2, wobei die Expression der zymogenen Vorstufe der Proteinase K durch Methanol induziert wird.

4. Verfahren gemäß einem der Ansprüche 1 bis 3, wobei die Sekretion des Proteins durch die N-terminale Fusionierung eines Signalpeptides eingeleitet wird

5. Verfahren gemäß einem der Ansprüche 1 bis 4, wobei die Sekretion des Proteins durch die N-terminale Fusionierung eines Signalpeptides des $\alpha$-Faktors aus Saccharomyces cerevisiae eingeleitet wird

6. Vektor enthaltend eine für die zymogene Vorstufe der Proteinase K kodierende DNA, wobei diese DNA stromaufwärts der kodierenden Sequenz mit einer Sequenz im Leserahmen fusioniert ist, welche für ein geeignetes Signalpeptid kodiert, und das kodierende Gen unter Kontrolle eines für die Wirtszelle geeigneten Promotors steht und dieser Vektor für die Transformation von Hefezellen geeignet ist, wobei die Hefezellen ausgewählt sind aus der Gruppe bestehend aus Pichia sp., Hansenula sp., Saccharomyces sp. und Schizosaccharomyces sp..

7. Vektor gemäß Anspruch 6, **dadurch gekennzeichnet, daß** er für die Transformation von Pichia pastoris geeignet ist.

8. Wirtszelle, ausgewählt aus der Gruppe bestehend aus Pichia sp., Hansenula sp., Saccharomyces sp. und Schizosaccharomyces sp., transformiert mit einem Vektor gemäß einem der Ansprüche 6 oder 7, wobei die Wirtszelle eine Hefe ist.

9. Wirtszelle gemäß Anspruch 8, wobei die Wirtszelle Pichia pastoris ist.

## Claims

1. Method for producing recombinant proteinase K comprising the steps:

   a) transformation of a host cell with a vector containing a DNA coding for the zymogenic precursor of proteinase K which is fused upstream of the coding sequence with a sequence in the reading frame which codes for a signal peptide and is under the control of a suitable promoter for the host cell,
   b) expression of the zymogenic precursor of proteinase K
   c) secretion and autocatalytic activation of proteinase K

   **characterized in that** the host cell is a yeast cell selected from the group comprising Pichia sp., Hansenula sp., Saccharomyces sp. and Schizosaccharomyces sp. and the protein is secreted in a soluble form by this expression host.

2. Method as claimed in claim 1, wherein the host cell is *Pichia pastoris.*

3. Method as claimed in one of the claims 1 and 2, wherein the expression of the zymogenic precursor of proteinase K is induced by methanol.

4. Method as claimed in one of the claims 1 to 3, wherein the secretion of the protein is initiated by the N-terminal fusion of a signal peptide.

5. Method as claimed in one of the claims 1 to 4, wherein the secretion of the protein is initiated by the N-terminal fusion of a signal peptide of the $\alpha$-factor from Saccharomyces cerevisiae.

6. Vector containing a DNA coding for the zymogenic precursor of proteinase K, wherein this DNA is fused upstream of the coding sequence with a sequence in the reading frame which codes for a suitable signal peptide, and the coding gene is under the control of a suitable promoter for the host cell and this vector is suitable for the transformation of yeast cells, wherein the yeast cells are selected from the group comprising Pichia sp., Hansenula sp., Saccharomyces sp. and Schizosaccharomyces sp.

7. Vector as claimed in claim 6, **characterized in that** it is suitable for the transformation of *Pichia pastoris.*

8. Host cell selected from the group comprising Pichia sp., Hansenula sp., Saccharomyces sp. and Schizosaccharomyces sp. transformed with a vector as claimed in one of the claims 6 or 7, wherein the host cell is a yeast.

9. Host cell as claimed in claim 8, wherein the host cell is *Pichia pastoris.*

**Revendications**

1. Procédé de préparation de protéinase K recombinante, comprenant les étapes suivantes :

a) transformation d'une cellule hôte à l'aide d'un vecteur qui contient un ADN codant pour le précurseur zymogène de la protéinase K, ce dernier étant fusionné, en amont de la séquence codante, avec une séquence dans le cadre de lecture, cette séquence codant pour un peptide signal et se trouvant sous le contrôle d'un promoteur approprié pour la cellule hôte,
b) expression du précurseur zymogène de la protéinase K,
c) sécrétion et activation auto-catalytique de la protéinase K,

**caractérisé en ce que** la cellule hôte est une cellule de levure choisie dans le groupe constitué par Pichia sp., Hansenula sp., Saccharomyces sp. et Schizosaccharomyces sp., et que la protéine est secrétée par cet hôte d'expression sous forme soluble.

2. Procédé selon la revendication 1, dans lequel la cellule hôte est Pichia pastoris.

3. Procédé selon l'une des revendications 1 et 2, dans lequel l'expression du précurseur zymogène de la protéinase K est induite par du méthanol.

4. Procédé selon l'une quelconque des revendications 1 à 3, dans lequel la sécrétion de la protéine est amorcée par la fusion N-terminale d'un peptide signal.

5. Procédé selon l'une quelconque des revendications 1 à 4, dans lequel la sécrétion de la protéine est amorcée par la fusion N-terminale d'un peptide signal du facteur $\alpha$ de Saccharomyces cerevisiae.

6. Vecteur contenant un ADN qui code pour le précurseur zymogène de la protéinase K, ce dernier étant fusionné, en amont de la séquence codante, avec une séquence dans le cadre de lecture, cette séquence codant pour un peptide signal approprié, et le gène codant est sous le contrôle d'un promoteur approprié pour la cellule hôte, et ce vecteur est approprié pour la transformation de cellules de levure, les cellules de levure étant choisies dans le groupe constitué par Pichia sp., Hansenula sp., Saccharomyces sp. et Schizosaccharomyces sp.

7. Vecteur selon la revendication 6, **caractérisé en ce qu'**il est approprié pour la transformation de Pichia pastoris.

8. Cellule hôte choisie dans le groupe constitué par Pichia sp., Hansenula sp., Saccharomyces sp. et Schizosaccharomyces sp., transformée à l'aide d'un vecteur selon l'une des revendications 6 ou 7, la cellule hôte étant une levure.

**9.** Cellule hôte selon la revendication 8, la cellule hôte étant Pichia pastoris.

Figur 1

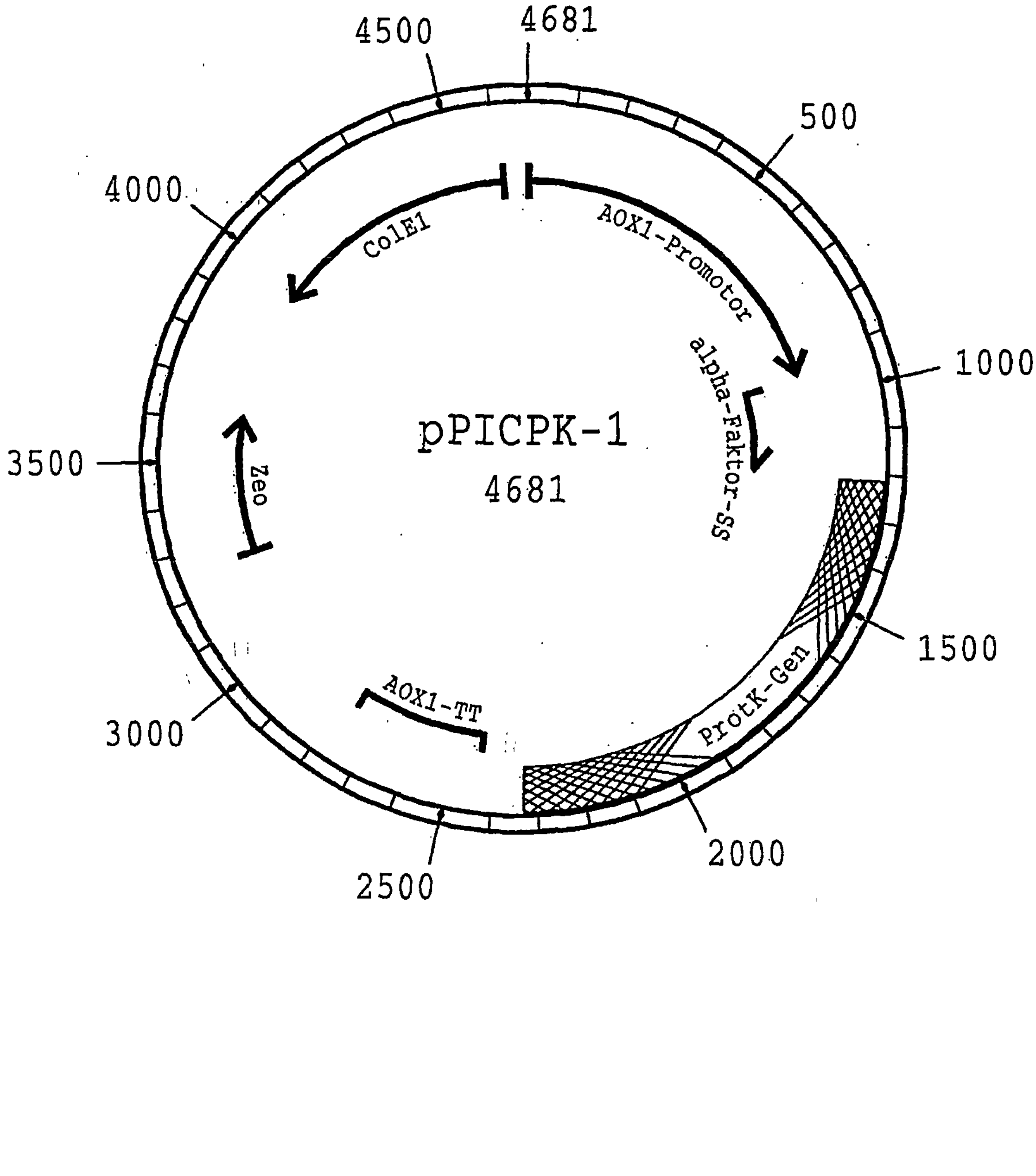
4500
4681
500
4000
1000
3500
1500
3000
2000
2500
ColE1
AOX1-Promotor
alpha-Faktor-SS
pPICPK-1
4681
Zeo
ProtK-Gen
AOX1-TT

Figur 2

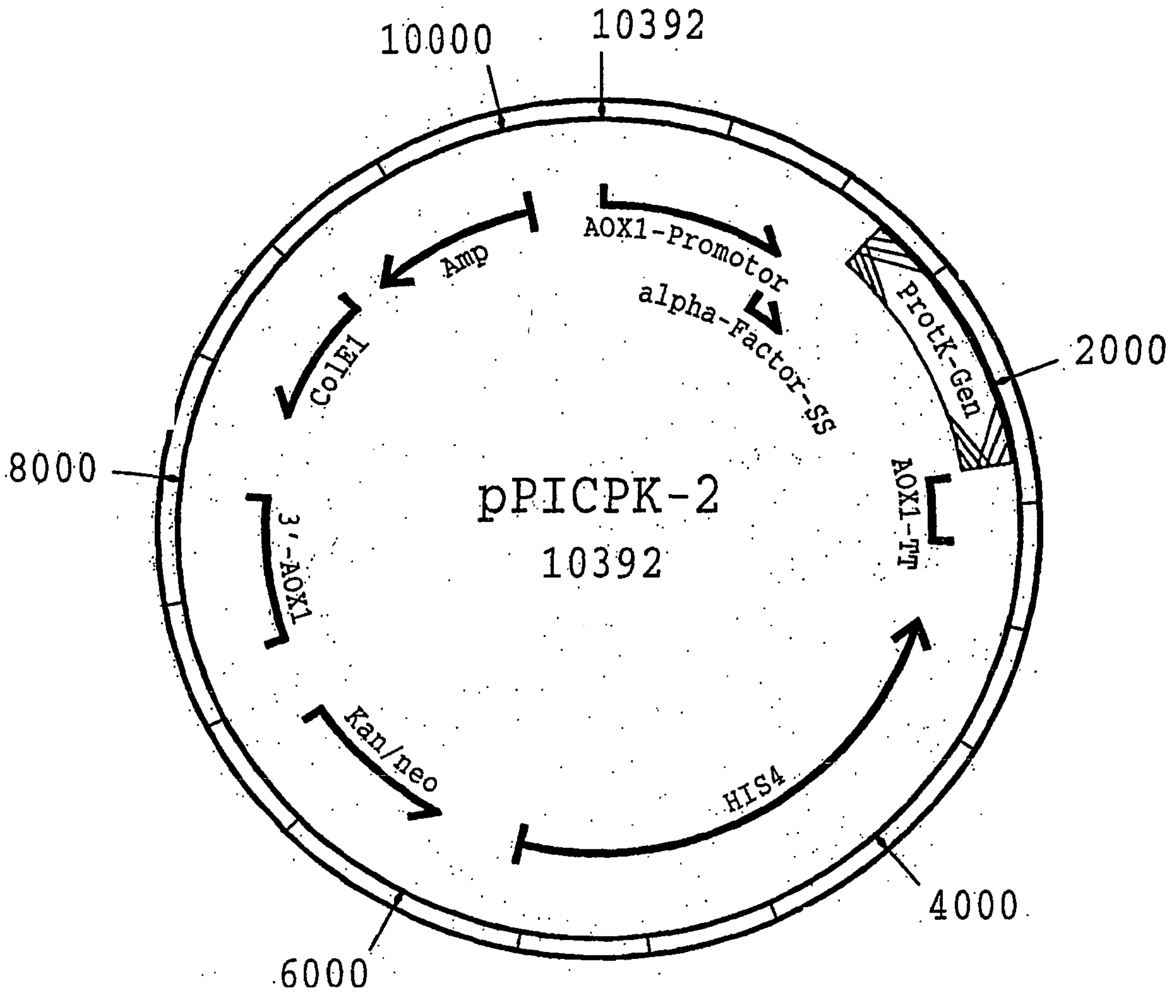
10000
10392
AOX1-Promotor
Amp
alpha-Factor-SS
ColE1
ProtK-Gen
2000
8000
pPICPK-2
10392
AOX1-TT
3'-AOX1
Kan/neo
HIS4
4000
6000